# EUROPÄISCHE PATENTSCHRIFT

(11) **EP 1 131 110 B1**
(45) Veröffentlichungstag und Bekanntmachung des Hinweises auf die Patenterteilung: **29.03.2006**
(21) Anmeldenummer: 99972113.7
(22) Anmeldetag: 16.11.1999
(51) Int. Cl.: A61L 24/10

(54) **STABILISIERTE PROTEINZUBEREITUNGEN FÜR EINEN GEWEBEKLEBER**
STABILISED PROTEIN PREPARATIONS FOR A TISSUE ADHESIVE
PREPARATIONS PROTEIQUES STABILISEES POUR ADHESIF TISSULAIRE

(30) Priorität: 18.11.1998 DE 19853033
(43) Veröffentlichungstag der Anmeldung: 12.09.2001
(73) Patentinhaber: ZLB Behring GmbH, 35041 Marburg (DE)
(72) Erfinder: METZNER, Hubert, D-35041 Marburg (DE); GRONSKI, Peter, D-35041 Marburg (DE)
(86) Internationale Anmeldenummer: PCT/EP1999/008812
(87) Internationale Veröffentlichungsnummer: WO 2000/029041

(56) Entgegenhaltungen:
- EP-A- 0 554 570
- EP-A- 0 804 933
- WO-A-94/00566
- US-A- 5 605 887
- CHABBAT ET AL: "Properties of a new fibrin glue stable in liquid state" THROMBOSIS RESEARCH, Bd. 76, Nr. 6, 15. Dezember 1994 (1994-12-15), Seiten 525-533, XP000884761

## Beschreibung

Gegenstand der Erfindung ist ein Gewebekleber sowie stabilisierte Proteinzubereitungen für einen Gewebekleber oder für parenteral applizierbare Präparate, die bei der Lagerung im flüssigen Zustand oder nach Lagerung im gefrorenen Zustand beim Wiederauftauen und bei weiterer Lagerung im flüssigen Zustand über mehrere Monate oder Jahre keinen wesentlichen Wirkungsverlust zeigen, dadurch gekennzeichnet, dass Faktor XIII und Fibrinogen getrennt vorliegen.

Es ist bekannt, dass zur Verbindung menschlicher oder tierischer Gewebe Gewebekleber eingesetzt werden können, die u.a. aus den Hauptkomponenten Fibrinogen, Faktor XIII und Thrombin bestehen. Diese Proteinpräparate bedürfen einer sorgfältigen Stabilisierung, damit ihre volle Wirkung und ihre Anwendungseigenschaften bis zu ihrem chirurgischen Einsatz bei der Gewebeklebung erhalten bleiben.

Die Gewebeklebung ist eine Methode, die schon zu Beginn dieses Jahrhunderts und danach immer wieder beschrieben wurde (S. Bergel: Über Wirkungen des Fibrins. Dtsch. med. Wschr. 35:663-5 (1909); E.P. Cronkite, E.L. Lozner, J.M. Deaver: Use of thrombin and fibrinogen in skin grafting. JAMA 124:976-8 (1944); H. Matras; H.P. Dinges, H. Lassmann, B. Mammoli, Wr. med. wschr. 37:517 (1972); H. Matras, H.P. Dinges, H. Lassmann, B. Mammoli: J. max. fac. Surg. 1:37 (1973); H. Matras, F. Braun, H. Lassmann, H.P. Ammerer, B. Mammoli: Plasma clot welding of nerves (experimental report) J. max. fac. Surg. 1:236-4 (1973); H. Kuderna, H. Matras. Wiener klin . Wschr. 87:495-496 (1975)). Während anfangs noch Plasma oder Fibrinogen als Pulver eingesetzt wurden, wurde später gereinigtes Fibrinogen zum Beispiel in der Form von Kryopräzipitat verwendet.

Mit der kommerziellen Herstellung von Fibrinogen-/Faktor XIII-und Thrombin-Konzentraten seit den siebziger Jahren hat die Gewebeklebung erheblich an Bedeutung gewonnen und wird heute beispielsweise zur Nahtunterstützung, lokalen Hämostase, versiegelung von Körperhöhlen zur vermeidung von Liquorverlust sowie zur Wundversorgung eingesetzt. Die Gewebeklebung mit Fibrinklebern ist eine physiologische Methode und hat deshalb bezüglich ihrer Verträglichkeit und der Abbaubarkeit der Kleberkomponenten Vorteile gegenüber synthetischen Klebern.

Kommerziell verfügbar sind Gewebekleber entweder als Lyophilisate oder als eingefrorene Präparate. Nach der Rekonstitution bzw. nach dem Auftauen sind die Produkte jedoch nur wenige Tage in Lösung stabil, da z.B. bei den hochkonzentrierten Fibrinogen-/Faktor XIII-Lösungen eine Aggregation und somit eine Viskositätserhöhung oder eine (z.B. proteolytische) Inaktivierung erfolgt, die eine weitere Anwendung unmöglich macht.

Auch die bislang in der Literatur beschriebenen und noch nicht kommerziell verfügbaren Gewebekleber bestehen in der Regel aus eingefrorenen oder gefriergetrockneten Komponenten, die vor der Verwendung aufgetaut oder aufgelöst werden müssen. Um die Verarbeitung, die Löslichkeit, das Auftauen oder die Stabilität des Fibrinogenkonzentrats zu verbessern, ist im europäischen Patent 0 085 923, in der deutschen Patentanmeldung 196 17 369 und in der europäischen Patentanmeldung 0 856 317 der Einsatz von chaotropen Agentien oder generell die Löslichkeit von Proteinen verbessernden Zusätzen wie Arginin oder Harnstoff oder ihren Derivaten oder Derivaten von Benzol, Imidazol, Pyrazol, Furan, Thiazol und Purin beschrieben worden. Unter chaotropen Agentien sind hier solche Agentien zu verstehen, die die Wechselwirkung von Proteinen oder Teilen davon reduzieren bzw. destabilisieren und somit deren Aggregationstendenz verringern. Dabei ist von Bedeutung, die Stabilität der Komponenten wie Fibrinogen und Faktor XIII auch in Anwesenheit dieser chaotropen Agentien und unter den gewählten Bedingungen zu gewährleisten. Dies ist bei gefrorenen und nach dem Auftauen noch für mehrere Wochen und Monate flüssig zu lagernden Fibrinogen-/Faktor XIII-Konzentraten oder bei nur flüssig zu lagernden Fibrinogen-/Faktor XIII-Konzentraten bisher noch nicht gelungen.

Sowohl bei der Flüssiglagerung, besonders aber auch bei der Lagerung im gefrorenen Zustand ist bei den bislang hierfür beschriebenen Formulierungen der Verlust an F XIII-Aktivität so hoch, dass der F XIII-Gehalt in Anwesenheit wirksamer Mengen chaotroper Agentien oft nach wenigen Wochen oder Monaten deutlich abfällt, teilweise bis unter die Nachweisgrenze.

Bei Formulierungen entsprechend der europäischen Patentanmeldung 0 856 317 hat sich hier gezeigt, dass Tranexamsäure (AMCA), besonders auch in Anwesenheit von chaotropen Agentien wie zum Beispiel Arginin und von anorganischen Salzen, den F XIII-Gehalt im Verlaufe der Lagerung bei -20°C deutlich reduziert (Tab. 1b, Ansatz 1). Die Lagerung bei 4°C führt bei dieser Formulierung zu einem Anstieg der Viskosität (Tab. 1a, Ansatz 1), was eine längere Lagerung auch ausschließt. Somit sind diese Formulierungen in Hinblick auf die gleichzeitige Stabilität von Fibrinogen und F XIII als nicht-stabil zu bezeichnen. Auch im Falle von Formulierungen entsprechend DE 196 17 369 zeigen sich Probleme beim Erhalt der F XIII-Aktivität (siehe Tab. 1, Ansatz 2 und 3). EP 554 570 beschreibt eine stabile Fibrinogenlösung, die mindestens zwei Absorbtionsschritten unterworfen wird, und so länger gelagert werden kann.

Aus der europäischen Patentschrift 0 487 713 ist außerdem ein biologischer Kleber für menschliche oder tierische Gewebe bekannt, der in flüssiger Form bei niedriger Temperatur stabilisiert ist. Dies soll dadurch erreicht werden, dass die Fibrinogen enthaltende Zubereitung mindestens ein chaotropes Agens in einer Konzentration zwischen etwa 0,3 M und 1 M enthält und der Kleber bei der Lagertemperatur flüssig ist.

Typischerweise enthielt ein solches Fibrinogenkonzentrat etwa 4 mM Tri-Natriumcitrat, 240 mM Nacl, 80 mM -Aminocapronsäure (EACA), 2 4 0 mM Glycin, 1% Polysorbat, 0, 6 g/l Natriumcaprolat, 0,5 M Harnstoff ggf. 2.000 KIE/ml Aprotinin und einen pH 7,5. Die Stabilität wurde bereits nach 1 Monat bewertet, was für ein therapeutisches Präparat sehr kurz ist. Die F XIII-Aktivität wurde dabei nicht bestimmt. J. Chabbat et al. berichteten auch von einem Fibrinogenkonzentrat, das bei 4°C über 6 Monate im flüssigen Zustand stabil ist (J. Chabbat, M. Tellier, P. Porte und M. Steinbuch: Properties of a new fibrin glue stable in liquid state. Thromb. Res. 76: 525-533 (1994)). Dieses Konzentrat enthielt als chaotrope Zusätze 0,5 M Harnstoff oder 5% Arginin (= 0,29 M) neben anderen Formulierungsbestandteilen, typischerweise 60 mM/l NaCl, 20 mM/l EACA und 60 mM/l Glycin. Allerdings wurde auch hier nicht der F XIII-Gehalt getestet.

Diese in der europäischen Patentschrift 0 487 713 und in der Literatur beschriebenen, flüssigen Formulierungen zeichnen sich dadurch aus, daß die Aggregation (Polymerisation) und somit die Viskositätserhöhung der konzentrierten Fibrinogenkomponente bei Kühlschranktemperatur verhindert oder reduziert wird. Allerdings wird Faktor XIII, ein wesentlicher Bestandteil von Fibrinogenkonzentraten für Fibrinkleber, unter diesen Bedingungen mehr oder weniger stark inaktiviert. Bei den für eine Lagerung im gekühlten Zustand entsprechend der europäischen Patentschrift 0 487 713 bzw. der verwandten Publikation von Chabbat et al. [J.Chabbat, M.Tellier, P.Porte und M.Steinbuch: Properties of a new fibrin glue stable in liquid state. Thromb. Res. 76: 525-533 (1994)] vorgesehenen Formulierungen stellt die Instabilität von F XIII dementsprechend ein wesentliches Problem dar, das auch durch die vorgeschlagenen Formulierungen nicht gelöst wird (siehe Tabelle 1, Ansätze 4-5). Weiterhin liegt der Gehalt an chaotropen Agentien mit ca. 0,3 bis 1,0 M/l relativ hoch, was geringere Konzentrationen chaotroper Agentien wünschenswert erscheinen läßt (<0,3 M/l).

Es lässt sich also feststellen, dass bei der Untersuchung der Stabilität von Fibrinogen/Faktor XIII-Zubereitungen sowie der Viskosität verschiedener bekannter Fibrinogen/Faktor XIII-Zubereitungen im gekühlten Zustand (0 bis 10°C) oder im gefrorenen Zustand mit anschließender Lagerung im gekühlten Zustand (0 bis 10°C) gefunden wurde, dass die bislang beschriebenen Formulierungen zu keinen stabilen Protein-Zubereitungen führen. Entweder Fibrinogen oder Faktor XIII zeigen im Laufe der Lagerzeit erhebliche Aktivitätsverminderungen oder die Aggregation von Fibrinogen führt zu einem viskosen, nicht mehr applizierbaren Material (siehe Tab. 1, Ansätze 1 bis 5).

Es stellte sich deshalb die Aufgabe, flüssige und über mehrere Monate stabile oder gefrorene und nach dem Auftauen über mehrere Monate stabile Proteinzubereitungen zu entwickeln, in der Fibrinogen bzw. Faktor XIII über Monate oder Jahre ohne wesentlichen Wirkungsverlust stabilisiert sind.

Gelöst wird diese Aufgabe durch stabilisierte Proteinzubereitungen, die gegenüber dem Stand der Technik den Vorteil haben, dass nicht nur Fibrinogen, sondern auch Faktor XIII durch die Zusätze stabilisiert wird und dass der Gehalt an chaotropen Reagentien reduziert werden kann, und vor allem dass Fibrinogen und Faktor XIII getrennt formuliert und somit stabil erhalten werden.

Dies erfolgt dadurch, dass bei eingefrorenen und nach dem Auftauen noch mehrere Wochen oder Monate stabil zu haltenden Präparaten ein chaotropes Agens entsprechend der hier gegebenen Definition in geringerer Konzentration zur vermeidung der Fibrinogen-Aggregation eingesetzt wird, dass die Konzentration anorganischer Salze reduziert wird und dass ggf. ein Antifibrinolytikum sowie weitere übliche Zusätze und Puffersubstanzen verwendet werden.

Als Antifibrinolytikum können Aprotinin oder Lysin oder ε-Aminocapronsäure (EACA) oder p-Aminomethylbenzoesäure (PAMBA) oder deren physiologisch unbedenkliche Salze eingesetzt werden. Bei Untersuchungen zum Einfluss verschiedener Antifibrinolytika hat sich überraschenderweise gezeigt, dass Lysin, PAMBA oder EACA die F XIII-Aktivität nicht negativ beinflussen, während bei Tranexamsäure dies der Fall ist. Als weitere Stabilisatoren für F XIII können z.B. Natrium-Citrat, Aminosäuren und Zucker eingesetzt werden.

Beide Proteinzubereitungen, die Faktor XIII und Fibrinogen mit ihren jeweiligen Stabilisatoren enthalten, sind getrennt voneinander aufzubewahren und erst unmittelbar vor der Anwendung als Gewebekleber zusammen mit der thrombinhaltigen Zubereitung zu vermischen. Gegenstand der Erfindung ist ein Gewebekleber, der aus einer den stabilisierten Faktor XIII enthaltenden Lösung, einer das stabilisierte Fibrinogen enthaltenden Lösung sowie einer stabilisiertes Thrombin enthaltenden Lösung besteht, die getrennt voneinander in einer zur gemeinsamen Anwendung vorbereiteten Verpackungseinheit bereitgestellt werden. Dies hat auch den weiteren Vorteil, dass bei Bedarf das Verhältnis von Faktor XIII und Fibrinogen geändert und der jeweiligen Situation angepasst werden kann.

### A) Gefrorene oder lyophilisierte Konzentrate, die auch im flüssigen Zustand noch mehrere Wochen/Monate bei 0 bis 10°C gelagert werden können (siehe Tabelle 1)

Stabile, gefrorene Fibrinogenkonzentrate sind bekannt und beschrieben, wobei deren Stabilität nach dem Auftauen aber auf wenige Tage begrenzt ist. Die beschränkte Haltbarkeit des Fibrinogenkonzentrats ist u.a. darauf zurückzuführen, dass die Viskosität durch Aggregation des Fibrinogens alsbald ansteigt. Zwar kann eine niedrige Viskosität durch den Zusatz von aggregationsverhindernden, also chaotropen Verbindungen im flüssigen Zustand erhalten werden. Diese Agentien haben aber den Nachteil, dass sie im gefrorenen Zustand (zum Beispiel bei -20°C) zu einem Abfall der Faktor XIII-Aktivität führen. Der Verlust an F XIII-Aktivität tritt dabei in der Regel umso schneller ein, je höher die Konzentration an chaotropen Agentien ist.

Bei der Entwicklung der erfindungsgemäßen stabilisierten Proteinzubereitungen hat sich nun gezeigt, daß nicht jedes chaotrope Agens die Stabilität des Faktor XIII gleichermaßen beeinflusst und dass vor allem auch die anderen erfindungsgemäß zuzusetzenden Additive von erheblichem Einfluß auf die Faktor XIII-Stabilität und auf die von der Fibrinogenaggregation beeinflusste Viskosität sind. So ist Arginin bei gleicher Molarität wesentlich effektiver beim Verhindern der Fibrinogenpolymerisation bzw. Aggregation als Harnstoff. Außerdem wirken sich anti-fibrinolytische Zusätze wie die ε-Aminocapronsäure (EACA), die p-Aminomethylcyclohexancarbonsäure (AMCA) oder die p-Aminomethylbenzoesäure (PAMBA) sowie deren physiologisch unbedenkliche Salze auf die Fibrinogen-Aggregation und die F XIII-Stabilität aus. Besonders AMCA wirkt hier negativ auf die F XIII-Aktivität bei Lagerung im gefrorenen Zustand. Überraschenderweise verursacht EACA, obwohl von der chemischen Struktur dem AMCA sehr ähnlich, unter entsprechenden Bedingungen nicht den gleichen Faktor XIII-Abfall wie AMCA. Außerdem wurde gefunden, dass die F XIII-Aktivität bei gefrorenen Protein-Konzentraten in Anwesenheit bestimmter Konzentrationen chaotroper Agentien nicht vermindert wird, wenn auf den in derartigen Zubereitungen bisher üblichen Zusatz anorganischer Salze ganz oder weitgehend verzichtet wird. So wurde erfindungsgemäß eine Zubereitung entwickelt, in der Fibrinogen nach dem Einfrieren und Wiederauftauen für mindestens mehrere Wochen oder sogar Monate flüssig bleibt und die Aktivität erhalten wird, wenn diese Formulierung als eine die Fibrinogenaggregation verhindernde oder vermindernde Substanz eine chaotrope Verbindung in einer Menge von weniger als 0,28 Mol/l enthält. Insbesondere Arginin in einer Menge von etwa 2 Gewichtsprozent hat sich bewährt. Andere, leicht chaotrope Agentien wie Citrullin, Nicotinamid. Harnstoff o.a. oder Mischungen davon bzw. mit Arginin können in einer Menge von bis zu ca. 0,28 M, insbesondere von 0,1 bis 0.20 M eingesetzt werden. Außerdem können neben Antifibrinolytika auch wasserlösliche anorganische Salze in Konzentrationen von ≤100 mMol/l, insbesondere von ≤50 mMol/l, ganz besonders bevorzugt in Konzentrationen von ≤20 mMol/l zugesetzt sein.

In einer der erfindungsgemäßen Zubereitungen bleibt sowohl das Fibrinogen als auch der Faktor XIII sowohl bei der Lagerung im gefrorenen als auch im flüssigen Zustand mindestens mehrere Wochen/Monate stabil. Für die Stabilität günstig kann noch der Zusatz anderer Komponenten wie zum Beispiel Salze der Zitronensäure oder der Milchsäure oder eine oder mehrere Aminosäuren oder ein Mono- oder Disaccharid oder ein Zuckeralkohol oder eine ihrer Mischungen sein. Bei diesen Zusammensetzungen kann die erfindungsgemäße Zubereitung wieder eingefroren und aufgetaut werden bzw. nach Rekonstitution eines Fibrinkleberlyophilisates wieder eingefroren und im gefrorenen Zustand als stabiles Fibrinogen/F XIII-Präparat gelagert werden. Da das Wiedereinfrieren bei den handelsüblichen, für einen Gewebekleber eingesetzten, gefrorenen oder lyophilisierten Proteinzubereitungen nicht möglich ist, zeigt sich hierin ein weiterer Vorteil der erfindungsgemäßen Formulierungen. Diese Eigenschaft erleichtert den Umgang mit Lyophilisaten nach dem Rekonstituieren oder von gefroren gelagerten Präparaten, falls nicht die gesamte Menge auf einmal verbraucht werden kann.

Die folgenden Beispiele erläutern die Herstellung von Fibrinogen-, Fibrinogen/Faktor XIII-(Referenzzubereitungen) bzw. Faktor XIII-Konzentraten zur Untersuchung der Stabilitäten unterschiedlicher Formulierungen. Als Ausgangsmaterialien sind zum Beispiel aber auch Fibrinogen, F XIII oder Thrombin aus transgener bzw. rekombinanter Herstellung einsetzbar:

### Beispiel 1:

Ein Fibrinogenkonzentrat wurde aus Kryopräzipitat durch Fällung, Al(OH)₃-Adsorption, Virusinaktivierung und weitere Fällung hergestellt [siehe P. Fuhge, P. Gratz, H. Geiger. Moderne Methoden zur Herstellung von Gerinnungstherapeutika. Behring Inst. Mitt. 79:164-176, (1986)]. Zur Virusinaktivierung bzw. -Entfernung können unterschiedliche chemische oder physikalische Verfahren eingesetzt werden, die gegen umhüllte und/oder nicht-umhüllte Viren effektiv sind. Das Fibrinogenkonzentrat wurde durch Diafiltration und anschließende Ankonzentrierung auf die jeweilige Zusammensetzung sowie auf eine Fibrinogen-Endkonzentration von mehr als 15 mg/ml, bevorzugt auf mehr als 60 mg/ml eingestellt. Die Stabilität dieser Fibrinogen-Präparate wurde in Anwesenheit von 0,05% Natriumazid durch Lagerung bei der jeweiligen Temperatur und Prüfung relevanter Analysenparameter wie gerinnbarem Fibrinogen, F XIII-Aktivität, Viskosität, Proteinabbau durch SDS-PAGE etc. bestimmt.

### Beispiel 2:

Gereinigter Faktor XIII wurde aus einer F XIII-haltigen Plasmafraktion (Cohn-Fraktion I) hergestellt (H.E. Karges und R. Rapp: Production and virus safety of human F XIII concentrates. in: Factor XIII, eds. J. McDonagh, R. Seitz, R. Egbring, Schattauer, Stuttgart/New York (1993), S. 66-76). Diese F XIII-Lösung wurde nach Dialyse oder Diafiltration und ggf. Ultrafiltration mit den zu testenden Stabilisatoren versetzt und nach Sterilfiltration bei verschiedenen Temperaturen gelagert oder zur Aufstockung von Fibrinogenkonzentraten verwendet.

### Beispiel 3:

Wie in Beispiel 1 wurde ein Fibrinogenkonzentrat hergestellt und der F XIII-Gehalt durch Zugabe einer Faktor XIII-Lösung aufgestockt. Durch anschließende Dialyse und Ankonzentrierung auf ca. 60 mg/ml Fibrinogen und höher sowie 10 E/ml Faktor XIII und höher wurden die für die Stabilitätsuntersuchung verwendeten Zubereitungen hergestellt. Zur Verhinderung von Bakterienwachstum enthielten die Ansätze noch 0,05% Natriumazid oder wurden mit Filtern von 0,2 *µ*m Porengröße sterilfiltriert.

### Beispiel 4:

Das lyophilisierte Fibrinogenkonzentrat eines kommerziellen Fibrinklebers (Beriplast P) wurde in Wasser für Injektionszwecke oder in Aprotinin-Lösung auf einen Fibrinogengehalt von >15 mg/ml, bevorzugt auf >60 mg/ml rekonstituiert und gegen Mischungen unterschiedlicher Zusätze dialysiert. In Anwesenheit von 0,05% NaN₃ zur Vermeidung von Bakterienwachstum wurden die Fibrinogen/F XIII-Konzentrate gelagert und ihre Stabilität nach verschiedenen Zeiten bestimmt.

### Beispiel 5:

Aus Kryopräzipitat mit nachfolgender AL(OH)₃-Adsorption und virusinaktivierung wird ein Fibrinogenkonzentrat hergestellt, dessen F XIII-Konzentration ggf. durch Zugabe von gereinigtem F XIII aufgestockt wird. Dieses Konzentrat wird durch Diafiltration und anschließende Ankonzentrierung auf die jeweilige Zusammensetzung sowie auf eine Fibrinogen-Endkonzentration von mehr als 15 mg/ml, bevorzugt auf mehr als 40 mg/ml eingestellt. Zur Überprüfung der Lagerstabilität wird in Anwesenheit von 0,05% Natriumazid gelagert.

### Beispiel 6:

Entsprechend den o.g. Beispielen wurden Fibrinogen-/Faktor XIII-Konzentrate hergestellt, gegen unterschiedliche Formulierungspuffer dialysiert und lyophilisiert. Die resultierenden Lyophilisate wurden direkt auf Stabilität getestet oder nach Rekonstitution wurden die erhaltenen Lösungen bei Temperaturen von 0-10°C gelagert und deren Stabilität wie angegeben überprüft.

Die Stabilität von Fibrinogen-, F XIII- sowie Fibrinogen-/F XIII-Präparaten hergestellt entsprechend einem der Beispiele 1-6 wurde durch Lagerung bei der jeweiligen Temperatur und Testung relevanter Analysenparameter bestimmt. Ergebnisse dieser Testungen sind in Tabelle 1 bis 3 aufgeführt. Generell sind jedoch auch andere Methoden zur Herstellung von Fibrinogen oder Faktor XIII geeignet, die z.B. andere Reiningungsschritte enthalten können.

### B) Flüssig gelagerte Konzentrate enthaltend Fibrinogen- oder Fibrinogen/Faktor XIII-Konzentrat (siehe Tabellen 1 und 2)

Auch bei flüssigen Fibrinogen- bzw. Fibrinogen-/Faktor XIII-Konzentraten, die nicht eingefroren, sondern nur im gekühlten Zustand bei ca. 0 bis 10°C gelagert werden, muß die Aggregation und somit die Viskositätserhöhung durch den Zusatz von chaotropen Agentien kontrolliert werden. Im Allgemeinen fällt dadurch die Faktor XIII-Aktivität mehr oder weniger stark ab (vgl. Tab 1, Ansatz 4 und 5). Es wurde nun gefunden, dass die Viskositätserhöhung verhindert oder vermindert und der Faktor XIII-Abfall verringert werden kann, wenn die chaotrope Substanz in einer Menge von weniger als 0,28 Mol/l eingesetzt wird. Als geeignete chaotrope Substanzen haben sich z.B. Arginin, Guanidin, Citrullin, Nicotinamid und ihre Mischungen erwiesen, wenn sie in der vorstehend genannten Menge eingesetzt werden. Vorteilhaft ist es weiterhin, der Zubereitung noch ein Antifibrinolytikum zuzusetzen, wobei vor allem Aprotinin, Lysin, ε-Aminocapronsäure (EACA), p-Amino-methylbenzoesäure (PAMBA) oder eines ihrer physiologisch verträglichen Salze oder Derivate in Betracht kommen, sowie als weiteren Stabilisator der Fibrinogen- bzw. Fibrinogen-Faktor XIII-Zubereitung physiologisch verträgliche Salze organischer Carbonsäuren, insbesondere der Zitronensäure oder der Milchsäure und ggfs. eine oder mehrere Aminosäuren oder ein Mono- oder Disaccharid oder Zuckeralkohol. Damit ist es möglich, in Fibrinogenkonzentraten mit einem Fibrinogengehalt von mehr als 15 mg/ml, insbesondere von mehr als 60 mg/ml, beim Einsatz von chaotropen Agentien bis zu einer Menge von 0,28 M verbesserte Stabilitäten zu erzielen.

Werden Mischungen von Fibrinogen und Faktor XIII hergestellt, dann sichert die gleichzeitige Anwesenheit von chaotropen Agentien und den obengenannten Zusätzen oder Mischungen, dass die Zubereitungen gegenüber den bekannten Formulierungen verbesserte Stabilitätswerte sowohl für Fibrinogen als auch für F XIII erreichen. Diese Mischung aus Fibrinogen und F XIII kann zusammen mit einer Thrombin enthaltenden Zubereitung in einer zur gemeinsamen Anwendung als Gewebekleber vorgesehenen Verpackungseinheit bereitgestellt werden.

### C) Flüssige Konzentrate mit getrennter Aufbewahrung von Fibrinogen und F XIII (siehe Tabellen 1-3)

Es hat sich gezeigt, dass die Stabilität einer flüssigen, Fibrinogen und F XIII enthaltenden Zubereitung weiter verbessert werden kann, wenn Fibrinogen und Faktor XIII getrennt aufbewahrt und erst unmittelbar vor bzw. während der Anwendung miteinander gemischt werden. In diesem Falle wird das F XIII-Konzentrat unabhängig von Fibrinogen stabilisiert. Wie sich gezeigt hat, kann die im wesentlichen fibrinogenfreie Faktor XIII-Zubereitung durch Zusatz eines physiologisch verträglichen Salzes einer organischen Di- oder Tricarbonsäure, insbesondere der Zitronensäure und den Zusatz weiterer üblicher Stabilisatoren für F XIII in einer Menge von bis zu 10 Gewichtsprozent, besonders bis zu 5 Gewichtsprozent, sowie Mischungen davon für die Lagerung im flüssigen Zustand bei 0 bis 10°C oder 20 bis 25°C stabilisiert werden (siehe Tabelle 3). Als übliche Stabilisatoren für F XIII werden Mono- oder Disaccharide oder Zuckeralkohole und Aminosäuren aus der Gruppe Glycin, Glycylglycin, Alanin, Cystein, Histidin, Glutamin oder physiologisch verträgliche Salze der Glutaminsäure oder der Asparaginsäure oder ihre Mischungen vorteilhaft eingesetzt. Weiterhin können ggf. Zusätze zur Regelung der Osmolarität, des pH-Wertes oder andere übliche Stabilisatoren für F XIII zugesetzt werden. Das Fibrinogenkonzentrat wird, wie bereits oben erwähnt, stabilisiert.

Die getrennte Lagerung von Faktor XIII- und Fibrinogen-Zubereitungen bei 0 bis 10°C, die jeweils mit spezifischen Stabilisatoren versehen sind, erlaubt die Herstellung eines Fibrinklebers, der aus drei flüssigen, stabilen Komponenten, nämlich dem Fibrinogenkonzentrat, F XIII-Konzentrat und dem Thrombin-Konzentrat besteht. In dieser Form sind die Komponenten über lange Zeit haltbar und behalten ihre Aktivität bis sie unmittelbar vor oder während der Anwendung als Gewebekleber miteinander gemischt werden. Die hier angegebenen Formulierungen erlauben auch das Einfrieren der einzelnen Komponenten ohne signifikanten Aktivitätsverlust.

### Formulierungen enthaltend Fibrinogen und/oder F XIII:

Formulierungen analog dem Stand der Technik:
1. 0,1 Mol/l NaCl, 3 g/l Na₃-Citrat x 2 H₂O, 8 g/l Glycin, 0,09 Mol/l L-Arginin, 0,58 Mol/l AMCA, pH 7,4
2. 6 g/l Na₃-Citrat x 2 H₂O, 1% Glycin, 2% Nicotinamid, 1.000 KIE/ml Aprotinin, pH 7,5
3. 1,8 g/l Na₃-Citrat x 2 H₂O, 16,3 g/l Glycin, 0,36 g/l Triton, 8,1 g/l HSA, 0,2 Mol/l Nicotinamid, pH 7,3
4. 0,15 Mol/l NaCl, 0,29 Mol/l L-Arginin, 1.000 KIE/ml Aprotinin, pH 7,0
5. 0,15 Mol/l NaCl, 0,5 Mol/l Harnstoff, 1.000 KIE/ml Aprotinin, pH 7,0

**Erfindungsgemäße Formulierungen sowie Vergleichsansätze enthaltend Fibrinogen oder Fibrinogen/Faktor XIII:**
6. 6 g/l Na₃-Citrat x 2 H₂O, 0,12 Mol/l L-Arginin, pH 7,4
7. 6 g/l Na₃-Citrat x 2 H₂O, 0,12 Mol/l L-Arginin, 0,14 Mol/l Citrullin, pH 7,4
8. 6 g/l Na₃-Citrat x 2 H₂O, 0,095 Mol/l L-Arginin, 80 mMol/l EACA, pH 7,4
9. 6 g/l Na₃-Citrat x 2 H₂O, 0,12 Mol/l L-Arginin, 0,14 Mol/l Citrullin, 80 mMol/l EACA, pH 7,4
10. 6 g/l Na₃-Citrat x 2 H₂O, 0,12 Mol/l L-Arginin, 80 mMol/l EACA, pH 7,4 *
11. 0,1 Mol/l NaCl, 6 g/l Na₃.-Citrat x 2 H₂O, 0,24 Mol/l L-Arginin, 80 mMol/l EACA, pH 7,4
12. 0,1 Mol/l NaCl, 6 g/l Na₃.-Citrat x 2 H₂O, 0,24 Mol/l L-Arginin, 320 mMol/l L-Lys, pH 7,4
13. 6 mg/ml Na₃-Citrat x 2 H₂O, 0,12 Mol/l L-Arginin, 0,14 Mol/l Citrullin, 80 mMol/l EACA, pH 7,4
14. 3 g/l Na₃-Citrat x 2 H₂O, 0,24 Mol/l L-Arginin, 80 mMol/l EACA, pH 7,0
15. 0,15 M NaCl, 6 g/l Na₃-Citrat x 2 H₂O, 0,24 Mol/l L-Arginin, 1.000 KIE/ml Aprotinin, pH 7,0
16. 6 g/l Na₃-Citrat x 2 H₂O, 0,24 Mol/l L-Arginin x HCl, 80 mMol/l EACA, pH 7,5
17. 6 g/l Na₃-Citrat x 2 H₂O, 0,24 Mol/l L-Arginin x HCl, 80 mMol/l PAMBA, pH 7,2
18. 6 g/l Na₃-Citrat x 2 H₂O, 0,24 Mol/l L-Arginin x HCl, 8% Mannit, 80 mMol/l EACA, pH 7,5
19. 6 g/l Na₃-Citrat x 2 H₂O, 0,15 M Nacl, 2% Nikotinamid, 1.000 KIE/ml Aprotinin, pH 7,5
20. 0,15 Mol/l NaCl, 6 g/l Na₃-Citrat x 2 H₂O, 0,24 Mol/l L-Arginin, 320 mMol/l Lys, pH 7,5
21. 0,15 Mol/l NaCl, 6 g/l Na₃-Citrat x 2 H₂O 0,24 Mol/l L-Arginin, 80 mMol/l EACA, pH 7,5
22. 6 g/l Na₃-Citrat x 2 H₂O, 0,24 Mol/l L-Arginin, 320 mMol/l Lys, pH 7,0
23. 6 g/l Na₃-Citrat x 2 H₂O, 0,24 Mol/l L-Arginin, 80 mMol/l EACA, pH 7,0
24. 0,15 Mol/l NaCl, 6 g/l Na₃-Citrat x 2 H₂O, 0,24 Mol/l L-Arginin, 2% L-His, 1.000 KIE/ml Aprotinin, pH 7,5
25. 0,15 Mol/l NaCl, 6 g/l Na₃-Citrat x 2 H₂O, 0,24 Mol/l L-Arginin, 2% Saccharose, 1.000 KIE/ml Aprotinin, pH 7,5
* Ansatz hergestellt durch Rekonstitution des entsprechenden Lyophilisates mit Wasser für Injektionszwecke

### Erfindungsgemäße Formulierungen enthaltend F XIII als Einzelkomponente

26. 1,5 g/l Na₃-Citrat x 2 H₂O, 2,9 g/l NaCl, 3 g/l L-Arginin x HCl, pH 7,4
27. 6 g/l Na₃-Citrat x 2 H₂O, pH 7,4
28. 1,5 g/l Na₃-Citrat x 2 H₂O, 2,9 g/l NaCl, pH 7,4
29. 3 g/l Na₃-Citrat x 2 H₂O, 1% Gly, pH 7,4
30. 3 g/l Na₃-Citrat x 2 H₂O, 2% Mannit, 10 mMol/l L-His, pH 7,4
31. 6 g/l Na₃-Citrat x 2 H₂O, 2% Mannit, pH 7,4
32. 6 g/l Na₃-Citrat x 2 H₂O, 1% HSA, pH 7,4
33. 5 mMol/l EDTA, 50 mMol/l Tris x HCl, pH 7,4
34. 6 g/l Na₃-Citrat x 2 H₂O, 1% L-His, pH 7,4
35. 1,5 g/l Na₃-Citrat x 2 H₂O, 2,92 g/l NaCl, 50 mMol/l Glycylglycin, pH 7,4
36. 3 g/l Na₃-Citrat x 2 H₂O, 1% L-His, pH 7,4
37. 3 g/l Na₃-Citrat x 2 H₂O, 38 mMol/l Glycylglycin, pH 7,4

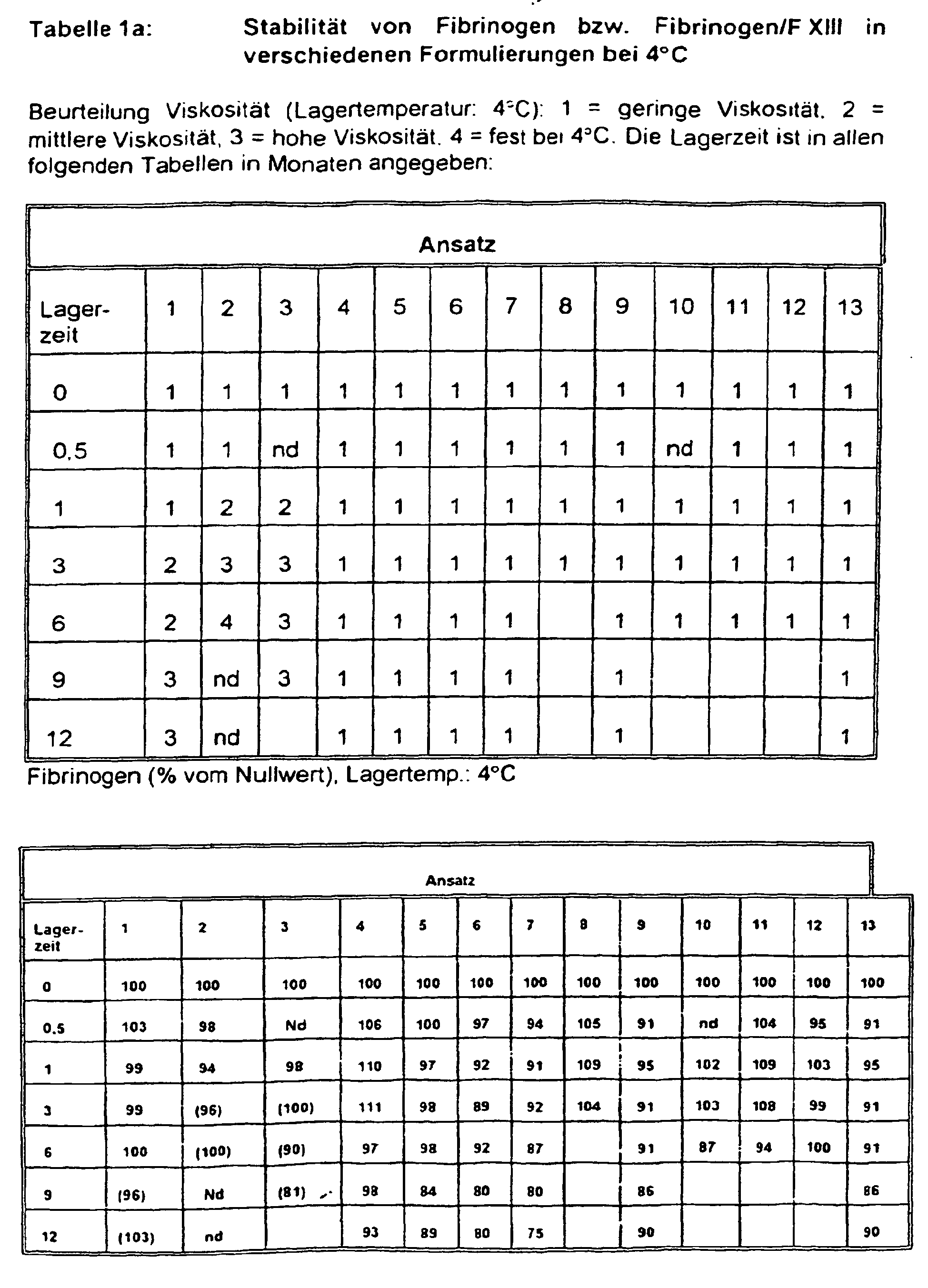

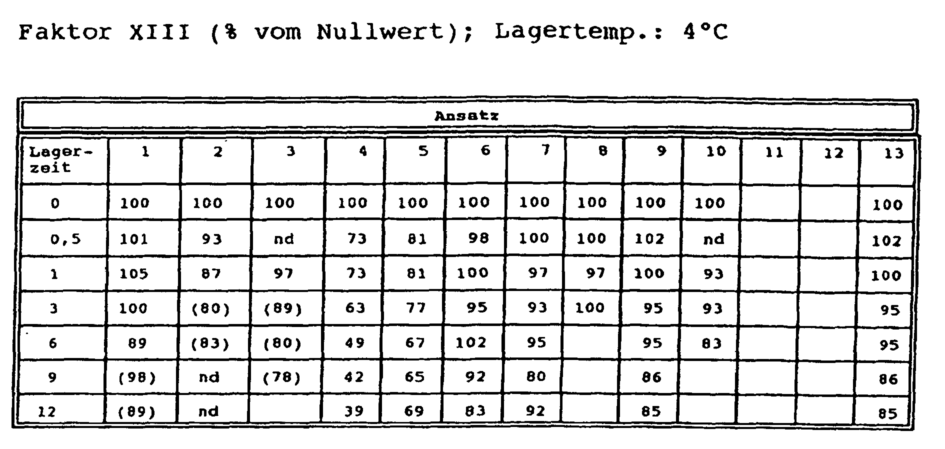

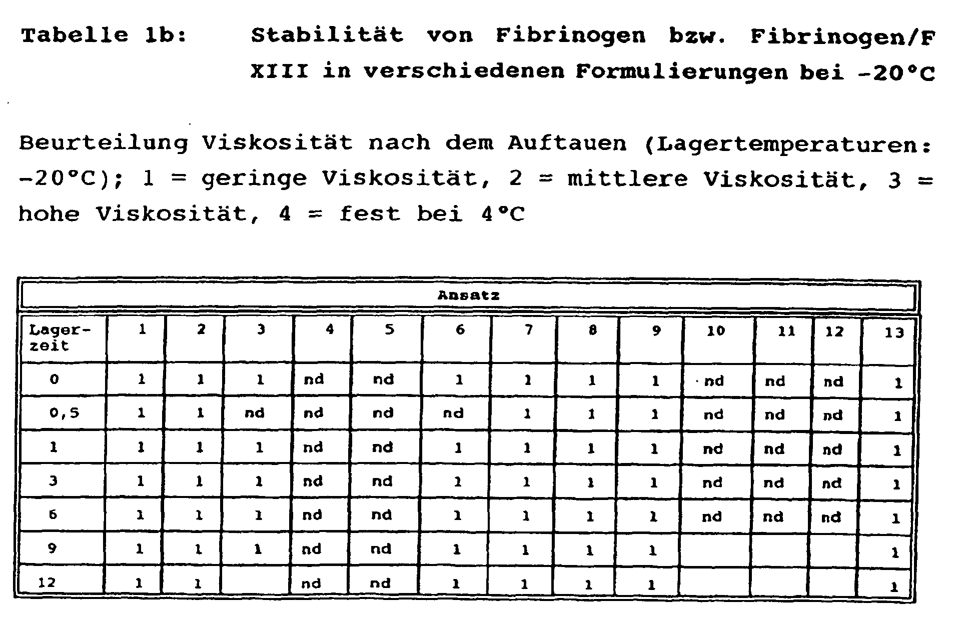

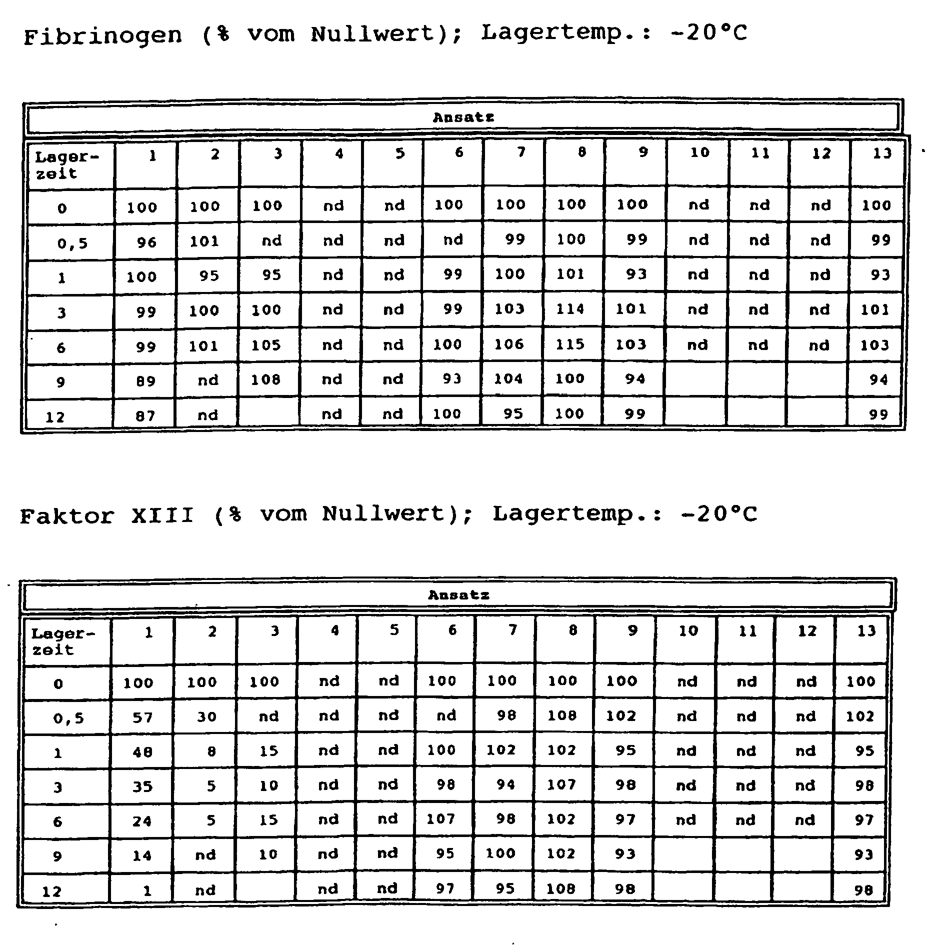

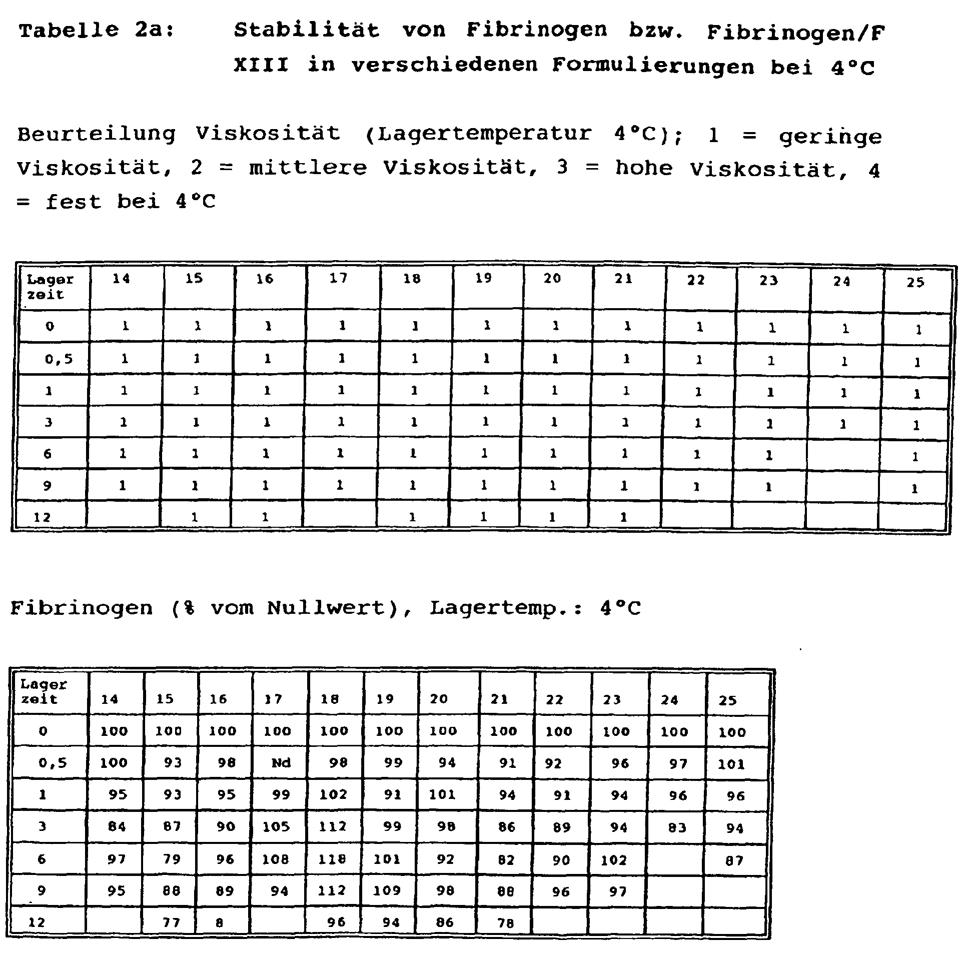

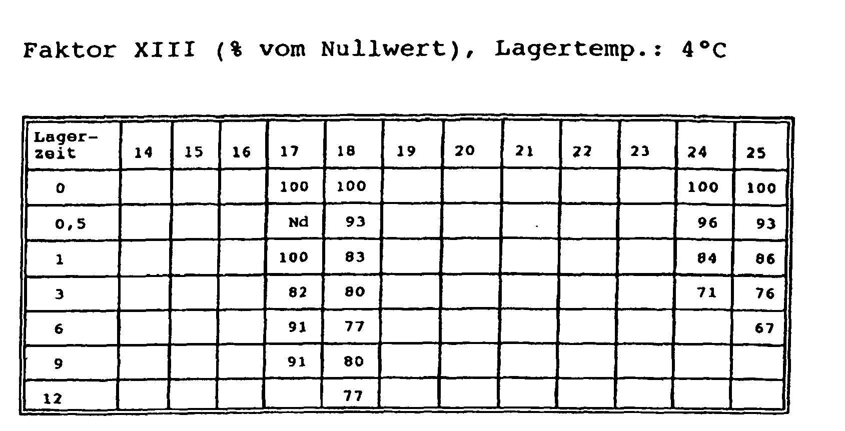

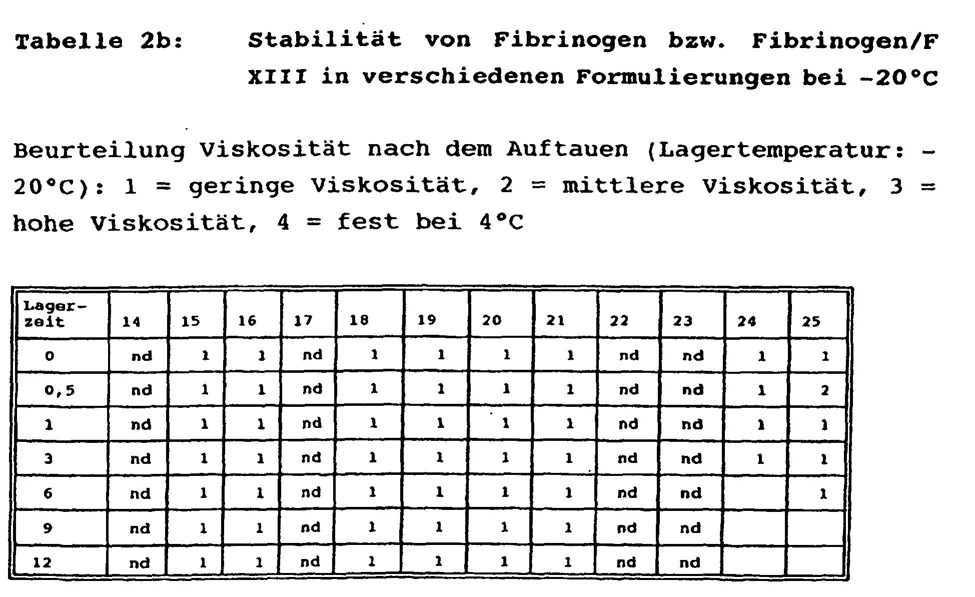

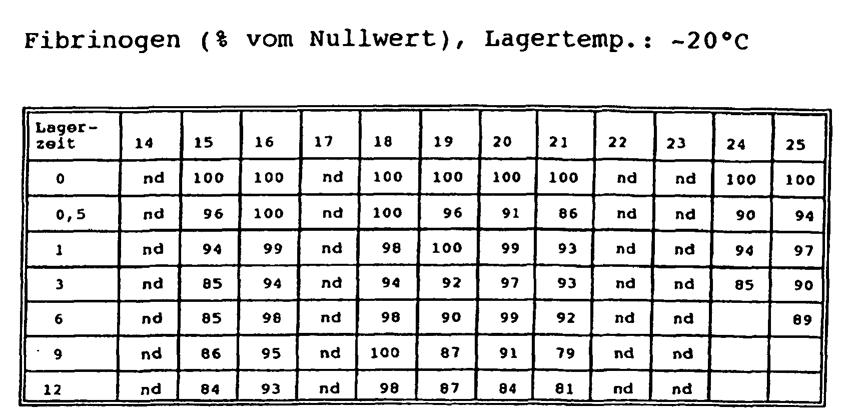

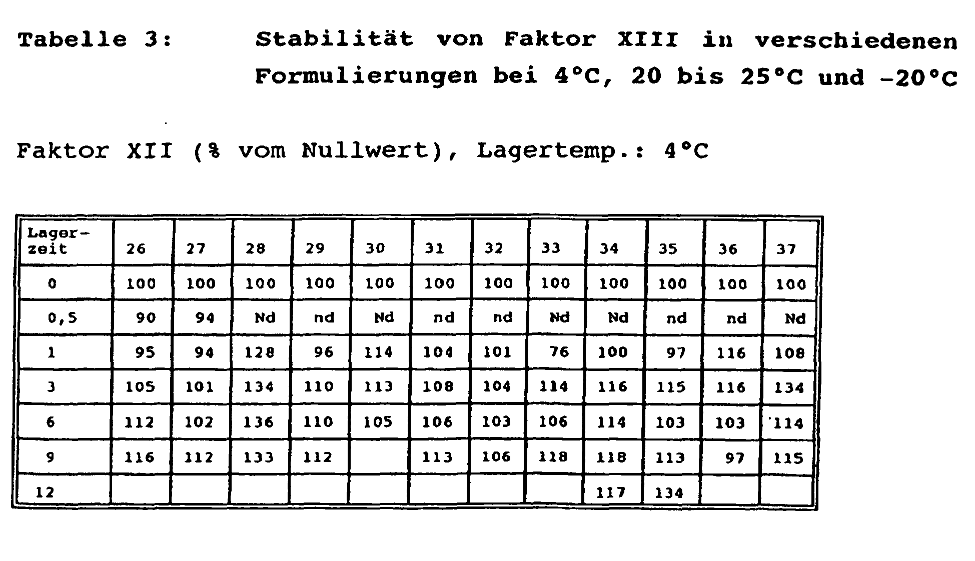

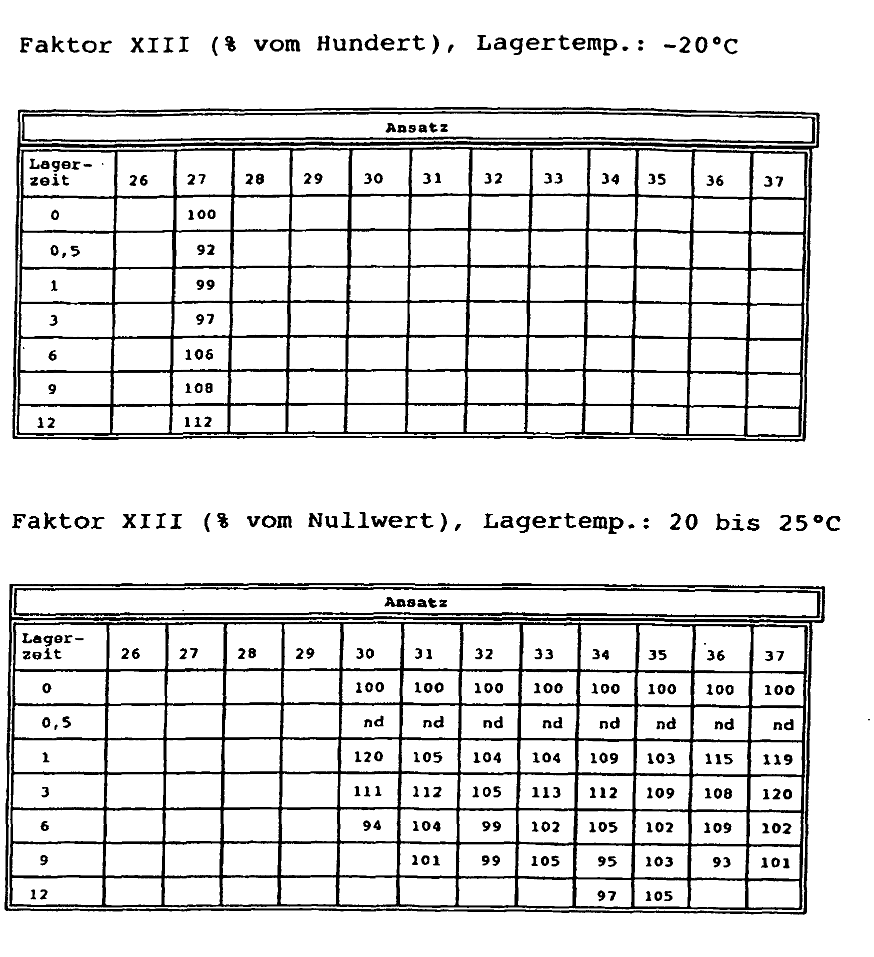

## Patentansprüche

1. Lagerfähiger Gewebekleber, **dadurch gekennzeichnet, dass** er drei voneinander getrennte Komponenten enthält, nämlich
(i) eine stabilisierte, im wesentlichen fibrinogenfreie, im flüssigen Zustand lagerfähige, den Blutgerinnungsfaktor XIII enthaltende Proteinzubereitung,
(ii) eine stabilisierte, im flüssigen Zustand lagerfähige, Fibrinogen enthaltende Proteinzubereitung, der zur Verhinderung oder Verminderung der Fibrinogenaggregation eine oder mehrere chaotrope Substanz(en) zugesetzt sind,
(iii) eine Thrombin enthaltende Zubereitung,
die in einer zur gemeinsamen Anwendung vorbereiteten Verpackungseinheit bereitgestellt werden.

2. Lagerfähiger Gewebekleber nach Anspruch 1, **dadurch gekennzeichnet, dass** der Fibrinogen enthaltenden Proteinzubereitung eine oder mehrere chaotrope Substanz(en) in einer Konzentration von weniger als 0,28 mol/l zugesetzt wird.

3. Gewebekleber nach Anspruch 1, **dadurch gekennzeichnet, dass** der den Faktor XIII enthaltenden und im wesentlichen fibrinogenfreien Proteinzubereitung
- ein physiologisch verträgliches Salz einer organischen Di-, Tri- oder Tetracarbonsäure, insbesondere der Zitronensäure, und
- weitere, teilweise bekannte Stabilisatoren und/oder Puffersubstanzen für den Faktor XIII zugesetzt sind.

4. Gewebekleber nach den Ansprüchen 1 bis 2, **dadurch gekennzeichnet, dass** der den Faktor XIII enthaltenden Proteinzubereitung als weitere Stabilisatoren
- ein Mono- oder Disaccharid oder ein Zuckeralkohol und/oder
- eine Aminosäure aus der Gruppe Glycin, Glycylglycin, Alanin, Cystein, Histidin, Glutamin oder ein physiologisch verträgliches Salz der Glutamin- oder Asparaginsäure und/oder
- ein reduzierendes oder oxidationsverhindemdes Agens und/oder
- eine oberflächenaktive Substanz zugesetzt sind.

5. Gewebekleber nach den Ansprüchen 1 bis 3, **dadurch gekennzeichnet, dass** der das Fibrinogen enthaltenden Proteinzubereitung zur Verhinderung oder Verminderung der Fibrinogenaggregation eine oder mehrere chaotrope Verbindungen aus der Gruppe bestehend aus Arginin, Guanidin, Citrullin, Harnstoff oder Derivate wie Nicotinamid oder ihren Mischungen zugesetzt ist

6. Gewebekleber nach den Ansprüchen 1 bis 4, **dadurch gekennzeichnet, dass** der das Fibrinogen enthaltenden Proteinzubereitung zusätzlich ein Antifibrinolytikum ausgewählt aus der Gruppe bestehend aus Aprotinin, ε-Aminocapronsäure (EACA), p-Aminomethylbenzoesäure (PAMBA), Lysin oder einem ihrer physiologisch verträglichen Salze zugesetzt ist.

7. Gewebekleber nach den Ansprüchen 1 bis 5, **dadurch gekennzeichnet, dass** der das Fibrinogen enthaltenden Proteinzubereitung zusätzlich als Stabilisator
- ein physiologisch verträgliches Salz einer organischen Carbonsäure, insbesondere der Zitronensäure oder der Milchsäure, oder
- eine oder mehrere Aminosäuren oder
- ein Mono- oder Disaccharid oder
- ein Zuckeralkohol oder
- anorganische Salze
oder eine ihrer Mischungen zugesetzt ist.

8. Verwendung der drei voneinander getrennten Komponenten eines Fibrinklebers gemäß einem der Ansprüche 1 bis 6 zur Herstellung eines Arzneimittels für die parenterale oder topische Anwendung.

9. Verfahren zur Zubereitung eines Gewebeklebers aus den drei voneinander getrennten Komponenten eines Fibrinklebers gemäß einem der Ansprüche 1 bis 6,
**dadurch gekennzeichnet, dass** unmittelbar vor der Verwendung als Gewebekleber die den Faktor XIII enthaltende Proteinzubereitung und
die das Fibrinogen enthaltende Proteinzubereitung
miteinander vermischt werden und dann während der Anwendung als Gewebekleber die Thrombin enthaltende Komponente hinzuzugeben ist.

10. Verfahren zur Zubereitung eines Gewebeklebers aus den drei voneinander getrennten Komponenten eines Fibrinklebers gemäß einem der Ansprüche 1 bis 6, **dadurch gekennzeichnet, dass** unmittelbar vor der Verwendung als Gewebekleber die den Faktor XIII enthaltende Proteinzubereitung und
die das Thrombin enthaltende Proteinzubereitung
miteinander vermischt werden und dann während der Anwendung als Gewebekleber die Fibrinogen enthaltende Komponente hinzuzugeben ist.

## Claims

1. A storable tissue adhesive comprising three components which are separate from one another, namely
(i) a stabilized protein preparation which is essentially fibrinogen-free and can be stored in the liquid state containing the blood clotting factor XIII,
(ii) a stabilized fibrinogen-containing protein preparation which is storable in the liquid state and to which one or more chaotropic substances have been added for preventing or decreasing fibrinogen aggregation,
(iii) a thrombin-containing preparation,
which are made available in a pack unit prepared for joint use.

2. The storable tissue adhesive as claimed in claim 1, wherein one or more chaotropic substances are added in a concentration of less than 0.28 mol/l to the fibrinogen-containing protein preparation.

3. The tissue adhesive as claimed in claim 1, wherein
- a physiologically tolerable salt of an organic di-, tri- or tetracarboxylic acid, in particular of citric acid, and
- further, partly known, stabilizers and/or buffer substances for factor XIII are added to the essentially fibrinogen-free protein preparation containing the factor XIII.

4. The tissue adhesive as claimed in claims 1 to 2, wherein
- a mono- or disaccharide or a sugar alcohol and/or
- an amino acid from the group consisting of glycine, glycylglycine, alanine, cysteine, histidine, glutamine or a physiologically tolerable salt of glutamic or aspartic acid and/or
- a reducing or oxidation-preventing agent and/or
- a surface-active substance
are added to the protein preparation containing the factor XIII as further stabilizers.

5. The tissue adhesive as claimed in claims 1 to 3, wherein one or more chaotropic compounds from the group consisting of arginine, guanidine, citrulline, urea or derivatives such as nicotineamide or their mixtures are added to the fibrinogen-containing protein preparation for preventing or decreasing fibrinogen aggregation.

6. The tissue adhesive as claimed in claims 1 to 4, wherein an antifibrinolytic selected from the group consisting of aprotinin, ε-aminocaproic acid (EACA), p-aminomethylbenzoic acid (PAMBA), lysine or one of their physiologically tolerable salts is additionally added to the fibrinogen-containing protein preparation.

7. The tissue adhesive as claimed in claims 1 to 5, wherein
- a physiologically tolerable salt of an organic carboxylic acid, in particular of citric acid or of lactic acid, or
- one or more amino acids or
- a mono- or disaccharide or
- a sugar alcohol or
- inorganic salts
or one of their mixtures are additionally added to the fibrinogen-containing protein preparation as stabilizer.

8. The use of the three mutually separate components of a fibrin adhesive according to any one of claims 1 to 6 in the manufacture of a medicament for parenteral or topical application.

9. A process for preparing a tissue adhesive from the three mutually separate components of a fibrin adhesive according to any one of claims 1 to 6, which comprises mixing the protein preparation containing the factor XIII and the fibrinogen-containing protein preparation immediately before use as a tissue adhesive and then the thrombin-containing component is to be added during the application as a tissue adhesive.

10. A process for preparing a tissue adhesive from the three mutually separate components of a fibrin adhesive according to any one of claims 1 to 6, which comprises mixing the protein preparation containing the factor XIII and the thrombin-containing protein preparation immediately before use as a tissue adhesive and then the fibrinogen-containing component is to be added during the application as a tissue adhesive.

## Revendications

1. Adhésif tissulaire pouvant être stocké, **caractérisé en ce qu'**il contient trois composants séparés les uns des autres, à savoir
(i) une préparation protéique stabilisée, essentiellement dépourvue de fibrinogène, pouvant être stockée à l'état liquide, contenant le facteur de coagulation sanguine XIII,
(ii) une préparation protéique stabilisée, pouvant être stockée à l'état liquide, contenant du fibrinogène, à laquelle on ajoute une ou plusieurs substance(s) chaotrope(s) pour inhiber ou réduire l'agrégation du fibrinogène,
(iii) une préparation contenant de la thrombine,
qui est proposée dans une unité de conditionnement prévue pour une utilisation conjointe.

2. Adhésif tissulaire pouvant être stocké selon la revendication 1, **caractérisé en ce que** l'on ajoute à la préparation protéique contenant le fibrinogène, une ou plusieurs substance(s) chaotrope(s) en une concentration inférieure à 0,28 moles/l.

3. Adhésif tissulaire selon la revendication 1, **caractérisé en ce que** l'on ajoute à la préparation protéique contenant le facteur XIII et essentiellement dépourvue de fibrinogène,
- un sel physiologiquement acceptable d'un acide di-, tri- ou tétracarboxylique, en particulier, de l'acide citrique, et
- d'autres stabilisants et/ou substances tampons, en partie connus, du facteur XIII.

4. Adhésif tissulaire selon les revendications 1 à 2, **caractérisé en ce que** l'on ajoute comme autres stabilisants à la préparation protéique contenant le facteur XIII,
- un mono- ou di-saccharide ou un polyol et/ou
- un acide aminé du groupe comprenant la glycine, la glycylglycine, l'alanine, la cystéine, l'histidine, la glutamine ou un sel physiologiquement acceptable de l'acide glutamique ou de l'acide aspartique et/ou
- un agent réducteur ou anti-oxydant et/ou
- une substance tensioactive.

5. Adhésif tissulaire selon les revendications 1 à 3, **caractérisé en ce que** l'on ajoute à la préparation protéique contenant le fibrinogène, pour inhiber ou réduire l'agrégation du fibrinogène, un ou plusieurs composé(s) chaotrope(s) du groupe consistant en l'arginine, la guanidine, 1a citruline, l'urée ou des dérivés tels que la nicotinamide ou leurs mélanges.

6. Adhésif tissulaire selon les revendications 1 à 4, **caractérisé en ce que** l'on ajoute à la préparation protéique contenant le fibrinogène, en outre un anti-fibrinolytique choisi dans le groupe consistant en l'aprotinine, l'acide ε-aminocaproïque (EACA), l'acide p-aminométhylbenzoïque (PAMBA), la lysine ou un de leurs sels physiologiquement acceptables.

7. Adhésif tissulaire selon les revendications 1 à 5, **caractérisé en ce que** l'on ajoute en outre comme stabilisant à la préparation protéique contenant le fibrinogène,
- un sel physiologiquement acceptable d'un acide carboxylique organique, en particulier de l'acide citrique ou de l'acide lactique, ou
- un ou plusieurs acides aminés ou
- un mono- ou di-saccharide ou
- un polyol ou
- des sels inorganiques ou un de leurs mélanges.

8. Utilisation des trois composants séparés les uns des autres d'un adhésif tissulaire selon l'une quelconque des revendications 1 à 6 pour la fabrication d'un médicament pour administration parentérale ou topique.

9. Procédé de préparation d'un adhésif tissulaire à partir des trois composants séparés les uns des autres d'un adhésif tissulaire selon l'une quelconque des revendications 1 à 6, **caractérisé en ce que**, immédiatement avant l'utilisation comme adhésif tissulaire, la préparation protéique contenant le facteur XIII et la préparation protéique contenant le fibrinogène sont mélangées l'une à l'autre, puis, pendant l'utilisation comme adhésif tissulaire, on ajoute le composant contenant la thrombine.

10. Procédé de préparation d'un adhésif tissulaire à base des trois composants séparés les uns des autres d'un adhésif tissulaire selon l'une quelconque des revendications 1 à 6, **caractérisé en ce que**, immédiatement avant l'utilisation comme adhésif tissulaire, la préparation protéique contenant le facteur XIII et la préparation protéique contenant la thrombine sont mélangées l'une à l'autre, puis, pendant l'utilisation comme adhésif tissulaire, on ajoute le composant contenant le fibrinogène.
